# EUROPEAN PATENT APPLICATION

(11) **EP 4 368 192 A1**
(43) Date of publication of application: **15.05.2024**
(21) Application number: 22206512.0
(22) Date of filing: 09.11.2022
(51) Int. Cl.: A61K 35/747, A61K 36/87, A61P 17/00, A61P 17/18, A61P 29/00, A61P 39/06, A61K 8/34, A61K 8/73, A61K 8/9789, A61K 8/99, A61Q 19/08

(54) **COMBINATION OF A LACTOBACILLUS STRAIN AND A GRAPE EXTRACT FOR USE AS AN ANTI-AGEING AGENT**

(71) Applicant: Cépages, 84350 Courthezon (FR)
(72) Inventor: BOUEZ, Sylvie, 03000 MOULINS (FR); DUBOURDEAUX, Alexandre, 03800 GANNAT (FR)
(74) Representative: Cabinet Laurent & Charras

(57) **Abstract**

The present invention concerns a composition comprising:
- an inactivated strain of lactobacillus; and
- an aqueous extract obtained from grape seeds and/or skins, and its use e.g. in cosmetics as an anti-ageing agent.

## Description

The present invention relates to an active ingredient to be used e.g. in cosmetics, comprising a lactobacillus strain and a grape extract.

It has been shown that such an ingredient displays a protective function versus the skin barrier, an antioxidant as well as an anti-inflammatory activity, and is therefore useful as an anti-ageing agent.

### BACKGROUND OF THE INVENTION

The skin, which main constituent is collagen, is the first interface between the human body and its environment, i.e. the interface between external and internal aggressions.

Ageing is not a disease, but a normal biological phenomenon that affects each of us in an unequal way. In addition to genetic factors, other factors amplify and even aggravate this natural phenomenon.

Today, all strategies to try to slow it down, from balanced nutrition to surgery, are possible challenges, but living and ageing well is still a playground for research.

To fully understand the effect of time on skin physiology, it is important to look at the two types of ageing, intrinsic and extrinsic. The intrinsic ageing is linked to genetics, whereas the extrinsic ageing is the one that many actors in the cosmetic and pharmaceutic fields are interested in.

Among the extrinsic factors, a wide variety of environmental factors such as solar ultraviolet (UV) radiations, visible light, environmental pollution, including ozone and particles, increase the production of reactive oxygen species (ROS) in the skin. In addition to reactive species induced by exogenous sources in the skin itself, some ROS are produced as by-products of oxidative metabolism in the mitochondria. In addition, the components of the skin microbiota (or microbiome or flora) can be affected by environmental factors such as UV and pollutants and are also linked to skin health. Finally, chronic psychological stress can also induce oxidative stress in the skin by decreasing the anti-toxic defenses.

Oxidative stress and the resulting oxidative damages can exacerbate pigmentation and skin ageing, leading to changes in the skin, such as tone evenness, wrinkles, sagging, dryness and roughness.

More generally, ageing is the result of an imbalance between the degradation and repair systems, e.g. in relation to the collagen metabolism, of a skin chronic inflammation but also of a loss of homeostasis of the skin tissue, notably at the level of the cutaneous microbiota.

Document FR 3 110 414 discloses a cosmetic composition for skin care comprising at least one ceramide, at least one tyndallized microorganism (e;g. *lactobacillus acidophilus* and/or *casei*) and at least one plant bisabolol.

However, there is still a need to provide further skin anti-ageing strategies.

### BRIEF SUMMARY OF THE INVENTION

As defined by the claims, the present invention relates to a composition combining a lactobacillus strain and a specific grape extract rich in polyphenols, especially tannins including crown tannins.

To the best knowledge of the Applicant, such a combination which numerous advantageous properties, useful in e.g. cosmetics, are reported in the present application has never been disclosed.

### Definitions

Unless otherwise defined, all technical and scientific terms used therein have the same meaning as commonly understood by one of ordinary skill in the art. The terminology used in the description is for the purpose of describing particular embodiments only and is not intended to be limiting.

The articles "a" and "an" are used herein to refer to one or to more than one (*i.e.,* to at least one) of the grammatical object of the article. By way of example, "an element" means one element or more than one element.

The terms "about" or "approximately" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 2, from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

As used herein, the term "compound", "ingredient", "component" or "agent" may be used interchangeably and designates a chemical or biological molecule which can be pure or mixed with other molecules. Said compound or ingredient or molecule can have an activity of interest, e.g. of cosmetic or therapeutic interest, and can therefore also be designated as an "active" or an "active principle". In case said compound or ingredient or molecule is merely used to formulate the compound of interest, it can be designated as an "excipient" or an "adjuvant". In relation to the composition of the invention, cosmetically or pharmaceutically acceptable excipients or adjuvants are preferably used.

### DETAILED DESCRIPTION OF THE INVENTION

According to a first aspect, the present invention concerns a composition comprising:
- an inactivated strain of lactobacillus; and
- an aqueous extract obtained from grapes.

According to a preferred embodiment, the strain of lactobacillus present in the composition belongs to the species *Lactobacillus gasseri* or *paragasseri,* advantageously *Lactobacillus paragasseri.*

According to a specific embodiment, the strain of lactobacillus is the strain deposited at the CNCM (Collection Nationale de Cultures de Microorganismes, Institut Pasteur, 25 rue du Docteur Roux, 75724 Paris Cedex 15) by LARENA (1, ZI du Taillis, Champtoceaux, F-49270 Orée d'Anjou) under number 1-5892 on September 13, 2022.

According to one embodiment, the strain of lactobacillus is inactivated by sterilization, advantageously by flash pasteurization or high-temperature short-time (HTST) processing, more advantageously in the following conditions:
- at a temperature comprised between 115°C and 140°C, preferably between 115°C and 130°C, for example at 124°C;
- for a time period between 15 and 60 seconds, preferably between 15 and 40 seconds, for example 30 seconds.

According to a specific embodiment, the inactivation of bacteria is not performed by tyndallization. As known in the art, a tyndallization process generally uses an intermittent sterilization method, i.e. cycles of heating (at temperatures below 60°C and for periods of up to 24 hours) and cooling the lactic acid bacteria. The aim of this process is to remove the spores and the bacteria from the tyndallized product.

Preferably, the strain is inactivated after mixture with the grape extract and possible further ingredients present in a composition according to the invention.

As a result, the living bacteria are stabilized by the grape extract and because of the adapted sterilization means, the composition of the invention contains a high percentage of intact bacteria. The bacterial fraction of the composition according to the invention is then different from bacterial biomass lysates or fermentation supernatants.

According to a preferred embodiment of the invention, the bacterial fraction represents between 0.1 and 2% w/w (weight/weight) of the composition, advantageously between 0.5 and 2% w/w or between 1 and 2% w/w.

Before inactivation, the composition advantageously comprises at least 10⁶ CFU (Colony Forming Unit) of bacteria per ml, more advantageously at least 10⁷ or 10⁸ or 10⁹ or even 10¹⁰ or 10¹¹ CFU/ml, for example 10⁹ CFU/ml.

After inactivation, the composition advantageously comprises at least 50% or 60%, more advantageously at least 70%, 80% or even 90% of intact bacteria. The percentage of intact bacteria can be measured by any technics known from the skilled person, e.g. by flow cytometry, to evaluate the membrane integrity of the bacteria.

According to the invention, the second component of the invention is a grape extract. According to one embodiment, the grapes implemented in the frame of the invention are fermented red grapes. According to one embodiment, the grape extract is obtained from grape seeds and/or skins, advantageously from grape seeds and skins. The grape seeds and/or skins can be obtained from pomace and separated mechanically.

The raw vegetal material, i.e. seeds and/or skins of grapes advantageously from fermented red wine grapes present in the pomace, is in the form of fresh matter possibly stored under cold conditions, advantageously under cold negative conditions, or dried by any technics known from the skilled person, e.g. under vacuum at 40-50°C.

According to another embodiment, the grape extract is an aqueous extract. In practise, the raw material is submitted to an aqueous extraction method which allows to solubilize a set of apolar substances of interest, especially polyphenols including tannins.

An aqueous extraction means that the grape material is incubated with an aqueous extraction solvent. Such a solvent can be pure water or a mixture of water with an alcohol in a minor proportion, e.g. with ethanol 20% volume/volume (V/V).

Typically, such an extraction is performed in the following conditions:
- at a temperature between 50°C and 80°C;
- for a time period between 1 and 2 hours, preferably between 1h30 and 2h;
- with a volume ratio (V/V) between the solvent and the material to be extracted between 1 and 10, preferably between 1 and 5, for example 4.2.

After incubation, the extraction product, i.e. the liquid crude extract, is preferably submitted to clarification by centrifugation (e.g. at 6000-11000 g), or filtration with e.g. a membrane having a cut off between 15 and 60 µm.

Advantageously, after extraction and possibly clarification or filtration, the dry matter (DM), including the molecules of interest especially tannins, represents at least 0.5% or even 1% of the extract.

The next stage is the purification of the extract to obtain tannin enrichment by membrane purification methods, advantageously ultrafiltration.

Typically, such an ultrafiltration is performed according to the following conditions:
- using a membrane having a cut off comprised between 10 and 200 kDa, preferably between 20 and 100 kDa, more preferably between 20 and 50 kDa;
- for a time period which can reach several hours.

Both aqueous extraction and ultrafiltration are aimed to concentrate especially procyanidolic oligomers and condensed trimeric, tetrameric and pentameric tannins including crown tannins.

The extract of interest corresponds to the retentate after ultrafiltration and typically comprises 40 to 50% of dry matter, including the molecules of interest, especially tannins.

Diafiltration using water can be implemented on the retentate.

The retentate is typically in a liquid form but can be dried by any technics known in the art, e.g. submitted to vacuum concentration, until the obtention of a dry extract.

According to one aspect, the present invention also concerns a method for obtaining an aqueous extract to be incorporated in a composition as claimed comprising:
- collecting seeds and/or skins, advantageously skins and seeds from grapes, more advantageously from fermented red grapes;
- submitting them to an extraction with water or an aqueous solvent;
- submitting the extract to ultrafiltration;
- collecting the retentate which corresponds to the aqueous extract of interest;
- possibly drying the retentate.

According to a preferred embodiment, the aqueous extract, to be incorporated in the composition of the invention, has at least one, advantageously all of the following characteristics:
- polyphenols in a total amount advantageously representing 2 to 5 % w/w of dry matter, e.g. 4%;
- tannins in a total amount advantageously representing 3 to 6 % w/w of dry matter, e.g. 5%;
- molecular tannins in an amount advantageously representing 0.05 to 0.15 % w/w of dry matter, e.g. 0.12%;
- crown tannins in an amount advantageously representing 0.005 to 0.015 % w/w of dry matter, e .g. 0.01%.

As known in the art, tannins are a specific type of polyphenols, i.e. molecules containing flavon-3-ol units: 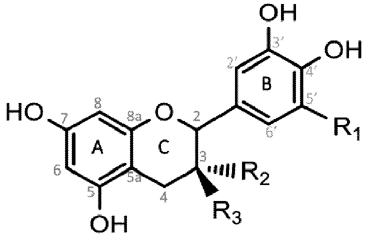 with

| | |
|---|---|
| 1. **(+1-Catéchine** | R1=H, R2=H, R3=OH |
| 2. **(-)-Épicatéchine** | R1=H, R2=OH, R3=H |
| 3. **(+)-Gallocatéchine** | R1=OH, R2=H, R3=OH |
| 4. **(-)-Épigallocatéchine** | R1=OH, R2=OH, R3=H |
| 5. **(-)-Épicatéchine-3-*O*-gallate** | R1=H, R2=O-acide gallique, R3=H |

Tannins can be present as monomers or as polymers. In the frame of the invention, molecular tannins (or condensed tannins) correspond to polymers of flavon-3-ol, including dimers, trimers, tetramers....

The so-called crown tannins include a tetramer having the following structure: 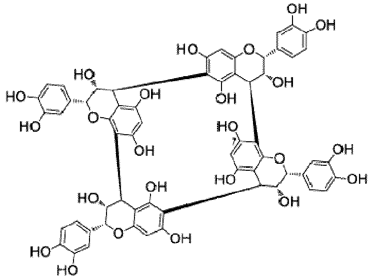 as well as pentamers having the following structure:

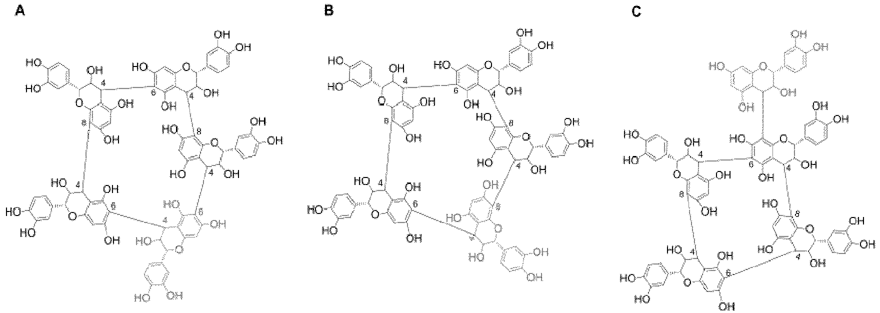

Methods for determining the nature and the amount of these different types of molecules are known in the art, as disclosed e.g. by Drinkine, J. et al. (J.Agric.Food Chem. 2007, 55(4), 1109-1116) and Lorrain, B et al. (Food Chem. 2011, 126, 1991-1999), and illustrated in the examples.

According to a still preferred embodiment, the aqueous extract, to be incorporated in the composition of the invention, has the following composition:
- tannins in a total amount advantageously representing 3 to 6 % w/w of dry matter, e.g. 5%;
- molecular tannins in an amount advantageously representing 0.05 to 0.15 % w/w of dry matter, e.g. 0.12%; and
- crown tannins in an amount advantageously representing 0.005 to 0.015 % w/w of dry matter, e .g. 0.01%.

Said amounts in the extract remain stable after incorporation of the other ingredients of the composition and inactivation of the bacteria.

According to another preferred embodiment, the aqueous extract represents between 4 and 8% w/w of the composition according to the invention.

Consequently, the composition according to the invention advantageously comprises:
- polyphenols in a total amount representing 0.08 to 0.4 % w/w of dry matter; and/or
- tannins in a total amount representing 0.12 to 0.48 % w/w of dry matter; and/or
- molecular tannins in an amount representing 0.002 to 0.012 % w/w of dry matter; and/or
- crown tannins in an amount representing 0.0002 to 0.0012 % w/w of dry matter.

More advantageously, the composition according to the invention comprises:
- tannins in a total amount representing 0.12 to 0.48 % w/w of dry matter;
- molecular tannins in an amount representing 0.002 to 0.012 % w/w of dry matter; and
- crown tannins in an amount representing 0.0002 to 0.0012 % w/w of dry matter.

According to another aspect, the present invention concerns a method of preparing a composition as claimed comprising:
- Mixing the aqueous extract with the lactobacillus strain; and
- Submitting the mixture to sterilization, advantageously flash pasteurization.

According to a preferred embodiment, the lactobacillus strain is cultivated under fermentative conditions and the bacteria are collected after centrifugation of the growth medium. Such a fraction, i.e. the pellet, is usually named bacterial biomass. It can be stored at -20-80°C.

Advantageously, said bacterial fraction typically comprises at least 10⁸ CFU (Colony Forming Unit) of bacteria per ml, more advantageously at least 10⁹ or 10¹⁰ or 10¹¹ or even 10¹² CFU/ml, for example between 10⁹ CFU/ml and 10¹¹ CFU/ml.

According to another embodiment, the other ingredients of the composition are added before the sterilization.

According to specific embodiments, the composition further comprises:
- water, advantageously representing between 45 and 70% w/w of the composition, more advantageously between 50 and 65% w/w;
- glycerin, advantageously representing between 15 and 40% w/w of the composition, more advantageously between 20 and 40% w/w;
- xanthan gum, advantageously representing between 0.05 and 0.3% w/w of the composition, more advantageously between 0.1 and 0.25% w/w.

According to another aspect, the invention concerns the use of a composition according to the invention in cosmetics or in medicine.

An example of a cosmetic composition according to the invention is as follows:

| **Component (INCI)** | **Range in % (w/w)** |
|---|---|
| Purified water | 45-65 |
| Composition of the invention (A) | 0,5-1,2 |
| Glycerin | 4-10 |
| Solagum AX (Acacia Senegal Gum (and) Xanthan Gum) | 0,2-0,5 |
| Arlacel 2121 (Sorbitan Stearate (and) Sucrose Cocoate | 4-8 |
| Montanov 68 (Cetearyl Alcohol (and) Cetearyl Glucoside) | 1-5 |
| Emogreen L 15 (C15-19 Alkane) | 4-8 |
| Dub MCT 5545 (CAPRYLIC/CAPRIC TRIGLYCERIDE) | 2-5 |
| Cetiol LC (Coco-Caprylate/Caprate) | 1-4 |
| Huile de Bourrache (Borago Officinalis Seed Oil) | 2-5 |
| Beurre de Karité (Butyrospermum Parkii (Shea) Butter) | 0-5 |
| Huile de Cameline (Camelina sativa oil ) | 0,5-4 |
| Coviox (Tocopherol) | 0,05-2 |
| Dermosoft 1388 (Glycerin (and) Aqua (and) Sodium Levulinate (and) Sodium Anisate) | 1-4 |
| Lactic acid (Lactic acid) | 0,01-1 |

According to a preferred embodiment, a composition according to the invention is for use as an anti-ageing agent, an antioxidant and/or an anti-inflammatory agent.

According to specific embodiments, a composition according to the invention is for use in the prevention or treatment of skin irritation and/or skin redness and/or skin hydration and/or skin barrier protection.

In relation to its ability to protect the skin barrier, the composition according to the invention can be used to prevent or even treat diseases linked to skin dysbiosis such as: atopic dermatitis (AD), psoriasis and hidradenitis suppurativa (HS).

In relation to its antioxidant activity, the composition according to the invention can be used to prevent or even treat cancers, especially skin cancer.

Depending on its application, the composition of the invention can be formulated for topical administration or for oral administration, and then used topically or orally.

In case of oral administration, e.g. when the composition of the invention is a nutraceutical, it can be in the form of a solid, liquid, solution, suspension, paste or gel.

According to one embodiment, it is in solid form, such as powder, tablets including effervescent tablets, pills, capsules or lozenges, prepared in a conventional manner using acceptable additives such as binding agents, filling agents, lubricants, disintegrating agents or wetting agents. The tablets may optionally be coated in a manner known to the person skilled in the art using sugars, films or enteric coatings.

Alternatively, other delivery systems may be used, such as preparations in soft gelatin or gelatin-based capsules, or formulations prepared using nanotechnology, such as nano-dispersions, nano-emulsions or nano-encapsulations.

Such a composition may further contain one or more additives such as colorants, pigments, or flavourings, ...

When a composition according to the invention is for cosmetic use, especially for topical application, it may be in the form of a cream, ointment, mask, serum, milk, lotion, paste, foam, aerosol, stick, deodorant, shampoo, conditioner, patch, aqueous-alcoholic or oily solution, oil-in-water (O/W) or water-in-oil (W/O) or multiple emulsion, aqueous or oily gel, liquid, pasty or solid anhydrous product, and/or oil dispersion in an aqueous phase using spherules possibly being polymeric nanoparticles such as nanospheres and nanocapsules or lipid vesicles of the ionic and/or non-ionic type.

A composition according to the invention may also comprise at least another cosmetically acceptable compound, preferably chosen from soothing agents, restructuring agents, regenerating agents, conditioning agents, sunscreens, antiwrinkle agents, moisturizing agents, anti-ageing agents, surfactants, fatty substances, organic solvents, solubilizing agents, thickening and gelling agents, smoothing agents, firmness-enhancing agents, elasticity and/or skin barrier effect, antioxidants, opacifiers, stabilizing agents, foaming agents, fragrances or perfumes, ionic or nonionic emulsifiers, fillers, sequestrants and chelators, filters, essential oils, colorants, pigments, hydrophilic or lipophilic active agents, lipid vesicles encapsulating one or more active agents and/or preservatives.

As examples, a composition according to the invention may comprise extracts of Reishi and/or apricot oil and/or jojoba seed oil and/or squalane and/or niacinamide and/or glycerin and/or fruit extracts of punica granatum and/or glyceril stearate and/or glyceryl stearate and/or alkanes of coconut and/or hemp oil and/or seed oil of Helianthus annuus, and/or seed oil of Simmondsia chinensis, and/or Pentylene glycol and/or radix root ferment filtrate and/or sclerotic gum and/or lysolecithin and/or caprate Coco-caprylate and/or maltodextrin, and/or xanthan gum and/or pullulan and/or butylene glycol and/or disodium cocoyl glutamate and/or lactic acid and/or sodium cocoyl glutamate and/or sodium hyaluronate and/or tocopherol and/or squalene and/or beta-sitosterol and/or pine bark extracts and/or red ginseng and/or white cigue (hemlock) and/or Cornus officinalis extracts and/or Boesenbergia rotunda extracts and/or N-acetylglucosamine and/or hyaluronic acid and/or konjac extracts and/or collagenase-digested AP peptide and/or pissenlita extracts.

The area of the skin to be treated can be the face or the body, advantageously the face, the neck and the neckline.

According to specific embodiments, the composition of the invention is:
- for moisturizing or hydrating the skin;
- for protecting or reinforcing the protection of the cutaneous barrier of the skin;
- for attenuating irritations of the skin;
- a skin soothing composition; and/or
- an anti-redness composition for the skin.

According to another aspect, the invention relates to a method for cosmetic care comprising a step of applying to an area of the skin the composition according to the invention.

Preferably, an amount of 0.1 to 50 mg/cm², preferably 0.25 to 2.5 mg/cm², of the cosmetic composition according to the invention is applied to the area of the skin.

Preferably, said application step is carried out on the face, the neck and the neckline.

The composition according to the invention may be applied once to twice a day, preferably 2 times a day, for at least 7 days, preferably at least 15 days, more preferably at least one month, particularly preferably at least 2 months, and more particularly preferably at least 3 months.

According to a particular embodiment, the cosmetic composition is applied in the method according to the invention 2 times a day for at least 3 months.

The practice of the present invention employs, unless otherwise indicated, conventional techniques of molecular biology (including recombinant techniques), microbiology, cell biology, biochemistry and immunology, which are well within the purview of the skilled artisan. Such techniques are explained fully in the literature, such as, "Molecular Cloning: A Laboratory Manual", fourth edition (Sambrook, 2012); "Oligonucleotide Synthesis" (Gait, 1984); "Culture of Animal Cells" (Freshney, 2010); "Methods in Enzymology" "Handbook of Experimental Immunology" (Weir, 1997); "Gene Transfer Vectors for Mammalian Cells" (Miller and Calos, 1987); "Short Protocols in Molecular Biology" (Ausubel, 2002); "Polymerase Chain Reaction: Principles, Applications and Troubleshooting" (Babar, 2011); "Current Protocols in Immunology" (Coligan, 2002). These techniques are applicable to the production of the polynucleotides and polypeptides of the invention, and, as such, may be considered in making and practicing the invention.

Particularly useful techniques for particular embodiments will be discussed in the sections that follow.

The disclosures of each and every patent, patent application, and publication cited herein are hereby incorporated herein by reference in their entirety.

Without further description, it is believed that one of ordinary skill in the art can, using the preceding description and the following illustrative examples, make and utilize the compounds of the present invention and practice the claimed methods.

### EXPERIMENTAL EXAMPLES

The invention is further described in detail by reference to the following experimental examples and the attached figures. These examples are provided for purposes of illustration only and are not intended to be limiting.

### FIGURES:

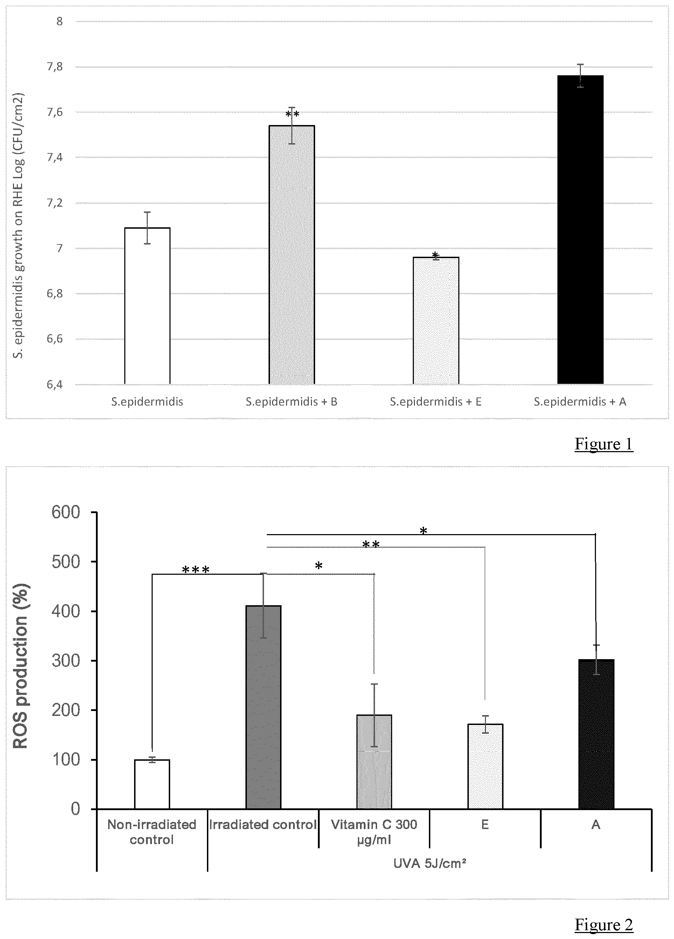

Figure 1: Booster effect of the composition according to the invention to protect the integrity of the skin barrier
   Growth of the commensal bacterium *Staphylococcus epidermidis* on a reconstructed epidermis model colonized by said bacterium after application of the extract alone (E) at 0.29% (w/w), the inactivated bacterium alone (B) at 0.079% (w/w) and then the combination of the two (A = E+B) at 0.3% (w/w).
   The histograms represent the values obtained from 3 replicates for each condition and are expressed as % of the untreated control. Statistical analysis (Student's t test) was performed comparing all conditions to untreated controls.
Figure 2: Effect of the grape extract alone (E) at 0.29% (w/w) and the composition according to the invention (A) at 0.3% (w/w) in comparison to a reference molecule (Vitamin C at 300 µg/ml) on the production of ROS in keratinocytes subjected to UVA 5J/cm².
   The histograms represent the values obtained from 3 replicates for each condition and are expressed as % of the untreated control. Statistical analysis (Student's t test) was performed comparing all conditions to untreated controls. *Values of 0.01<p<0.05 were considered as significant (*^{∗}*)*, *0.001<p<0.01 as highly significant (*^{∗∗}*)*, *and <0.001 as very highly significant (*^{∗∗∗}*)*.

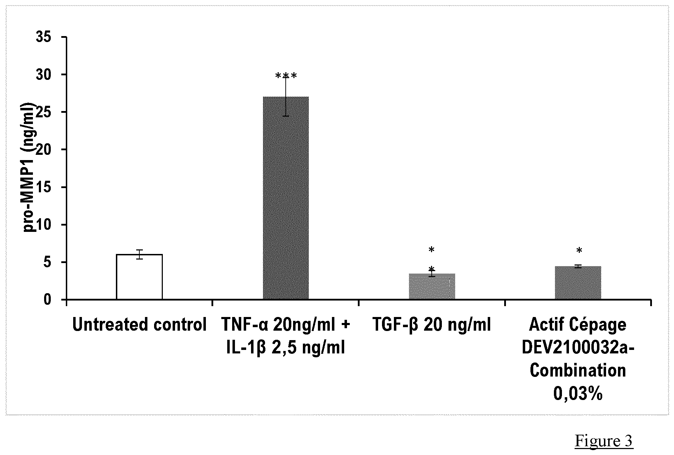

Figure 3: Pro-MMP1 abundance from NHDFs treated 72 hours by the active (A) of the invention.
   The histograms represent the values obtained from 3 replicates for each condition. Statistical analysis (Student's t test) was performed comparing all conditions to untreated controls. *Values of 0.01<p<0.05 were considered as significant (*^{∗}*)*, *0.001<p<0.01 as highly significant (*^{∗∗}*)*, *and <0.001 as very highly significant (*^{∗∗∗}*)*.

### I/ PREPARATION OF A COMPOSITION ACCORDING TO THE INVENTION

### I-1/ PREPARATION OF THE BACTERIA (B)

### Strain:

The bacterial strain used in the examples is the strain of the species *Lactobacillus paragasseri* deposited at the CNCM (Collection Nationale de Cultures de Microorganismes, Institut Pasteur, 25 rue du Docteur Roux, 75724 Paris Cedex 15) by LARENA (1, ZI du Taillis, Champtoceaux, F-49270 Orée d'Anjou) under number 1-5892 on September 13, 2022.

### Growth conditions

### Medium :

An adequate medium for culturing the strain under fermentative conditions has e.g. the following composition (Table 1):

| **Component** | **Concentration (g/l)** |
|---|---|
| Yeast extract | 40 - 80 |
| Glucose | 20-50 |
| Polyoxyethylenesorbitan monooleate | 03 - 07 |
| Di potassium hydrogen phosphate | 0,5 - 03 |
| Sodium acetate | 01 - 07 |
| Amonium citrate dibasic | 0,5 - 04 |
| Manganese sulfate | 0 - 1,05 |
| Magnesium sulfate | 0 - 2,02 |
| Deionised water | QSP |

### Conditions

The strain has been cultivated under classical fermentative conditions.

The bacteria were collected by centrifugation (5000g for 10 minutes) and the pellet fraction typically comprises 10⁸ to 10¹³, usually 10¹⁰ to 10¹² CFU (or bacteria)/ml.

### 1-2/ PREPARATION OF THE GRAPE EXTRACT (E)

An extract containing the molecules of interest, especially tannins, has been obtained from different varieties and different parts of the grapes, i.e. skins and seeds of fermented red grapes isolated from pomace.

The raw material was then submitted to an aqueous extraction, mixing it with 4.2 (w/w) part of water at a temperature of 50°C to 80°C for 1h30 to 2h.

The extract was then submitted to clarification by filtration using a membrane with a cut off of 50 µm. At this stage, the dry matter (DM) of the extract expressed in weight/weight (w/w) was estimated at 0.9% as measured on a probe of 3-5 g at a temperature of 110-115°C for several minutes in a desiccator (Mettler Toledo).

In a next stage, the extract was submitted to ultrafiltration in order to concentrate the molecules of interest. In practice, ultrafiltration using a membrane having a cut off of 10-50 kDa was performed.

The extract of interest corresponds to the retentate after ultrafiltration typically comprising 40 to 50% of dry matter, whereas the permeate containing the rest of the dry matter is discarded.

Water was used for diafiltration and the retentate was then submitted to vacuum concentration until the obtention of the extract (E).

The composition of such an extract was determined using the following protocols:

### Reagents and Standards

Deionized water was purified with a Milli-Q water system (Millipore, Bedford, MA, USA). HPLC grade acetonitrile, ethyl acetate, chloroform, methanol, ethanol and acetone purchased from Scharlau (Sentmenat, Barcelona, Spain). The following chemicals were obtained from Sigma Aldrich (St. Louis, MO, USA): (+)-catechin, (-)-epicatechin, B1 [(-)-epicatechin-(4β-8)-(+)-catechin], procyanidin dimer B2 [(-)-epicatechin-(4β-8)-(-)-epicatechin], gallic acid.

The Laboratory of Organic Chemistry and Organometallic (Université Bordeaux 1) synthesized procyanidins dimers B3 [(+)-catechin-(4α-8)-(+)-catechin], B4 [(+)-catechin-(4α-8)-(-)-epicatechin] and a trimer (C1) [(+)-catechin-(4β-8)-(+)-catechin-(4β-8)-(-)-epicatechin] [55].

### Grape extract analysis

### Tannins Extraction

Grape extract Alone (E) (1 g of dry matter) was solubilized in water/ethanol (250 mL, 95:5, v/v) and partitioned three times with chloroform (250 mL) to remove lipophilic material. The aqueous phase was then extracted three times with ethyl acetate (250 mL) to obtain two distinctive fractions: a low molecular weight procyanidin fraction (monomeric/oligomeric tannins) in the organic phase and a high weight procyanidin fraction (polymeric tannins) in the aqueous phase. These two fractions were concentrated and lyophilized.

### Polyphenols and Total Tannins

Crude extracts were solubilized in water/ethanol (90:10, v/v; pH 3.5 with tartaric acid) at appropriate concentrations. Polyphenol content was measured using Folin-Ciocalteu reagent as disclosed by Singleton and Rossi (Am J Enol Vitic. 1965, 16(3), 144-158). Total tannin content was measured by acidic hydrolysis using the method of Ribereau-Gayon and Stonestreet (Chim. Anal. 1966, 48, 188-196).

### Determination of Individual Tannins by HPLC Analysis

The extract was solubilized in a methanol/water solution (50:50, v/v) at appropriate concentrations and analyses of monomeric/oligomeric tannins (catechin, epicatechin, dimers B1, B2,B3,B4 ; Trimer C2) were carried out according to the method of Silva et al. (Eur. Food Res. Technol. 2012, 234, 361-365). The data was expressed as catechin equivalent/g dry weight GPE.

### Determination of Mean Degree of Polymerization (mDP)

The proanthocyanidin mean degree of polymerization (mDP) was determined for GPE in monomeric/oligomeric and polymeric tannin fractions by the means of phloroglucinolysis (Drinkine, J. et al., J.Agric.Food Chem. 2007, 55(4), 1109-1116). Analyses were carried out using the same method as described by Lorrain et al. (Food Chem. 2011, 126, 1991-1999).

The oligomeric and polymeric proanthocyanidins were depolymerised in the presence of a nucleophilic agent phloroglucinol in an acidic medium. Reversed-phase HPLC analysis of the products formed allows determination of the structural composition of proanthocyanidins, which are characterised by the nature of their constitutive extension units (released as flavan-3-ols phloroglucinol adducts) and terminal units (released as flavan-3-ols). To calculate the apparent mDP, the sum of all subunits (flavan-3-ol monomer and phloroglucinol adducts, in mols) was divided by the sum of all flavan-3-ol monomers (in mols). GPE sample was analysed with a Surveyor series instrument (Thermo-Finnigan, Les Ullis, France) equipped with a 100 x 4.6 mm i.d., 3.5 µm X-Terra reversed-phase C18 column (Waters) thermostated at 25°C. Detection was carried out at 280 nm using a Finnigan Surveyor PDA Plus detector. The mass detection was carried out using a Finnigan LCQ DECA XP MAX mass spectrometer with an ESI interface, performed in positive mode with the following parameters: capillary temperature 325 _C, capillary voltage 4 V, nebulizer gas flow 1.75 L/min, desolvation gas flow 1 L/min, and spray voltage 5 kV. The solvents used were solvent A: H2O/AcOH (99:1 v/v), and B: MeOH. The gradient consisted of 5% B during 25 min, linear gradient 5%-20% B in 20 min, then 20%-32% B in 15 min, finally 32%-100% B in 2 min. The column was washed with 100% B for 5 min and then stabilized with the initial conditions for 10 min. The injection volume was 20 µL. The flow rate was 1 mL/min.

### Determination of crown tannins in E

The UPLC-MS system used was an Agilent 1290 Infinity equipped with an ESI-Q-TOF-MS (Agilent 6530 Accurate Mass). The chromatographic separation was performed on an Eclipse Plus C18 column (2.1 × 100 mm, 1.8 µm). The solvents used were: water with 0.1% formic acid for solvent A and methanol with 0.1% formic acid for solvent B at a flow rate of 0.3 ml/min. The gradient of solvent B for the analysis was as follows: 6% B for 80.5 min; 6-95% B in 13.5 min; 95% B for 4 min and the UPLC column was equilibrated for 3 min using the starting condition. The ESI conditions were as follows: gas temperature and flow rate were set at 300°C and 9 L/min respectively; jacket gas temperature and flow rate were set at 350°C and 11 L/min respectively; capillary voltage was set at 4000 V. The fragmenter was always set at 200 V. The data obtained was processed using Mass Hunter Qualitative Analysis software. A calibration curve using the previously purified procyanidinic crown tetramer was established for the quantification of the procyanidinic crown tetramer and the procyanidinic crown pentamer. The results are expressed as mg crown tetramer equivalent/g dry matter.

The result is shown in Table 2 below:

| | | | | |
|---|---|---|---|---|
| % of dry matter (DM) of the extract | Total Polyphenols | Total Tannins | Molecular Tannins | Crown Tannins |
| | 2,1 | 2,5 | 0,06 | 0,0055 |

### 1-3/ PREPARATION OF THE COMPOSITION ACCORDING TO THE INVENTION (A = B+E)

An active according to the invention (A) can have the following composition (Table 3):

| **Component in the composition of the invention(A)** | **% (w/w ?)** |
|---|---|
| Purified water | 59.3 |
| **B:** *Lactobacillus paragasseri* (1-5892) inactivated by flash pasteurization | 0.5 |
| **E:** Dry extract of Grape pomace (*Vitis vinifera L.*) | 5 |
| Vegetable glycerin (E422) | 35 |
| Xanthan Gum (E415) | 0.20 |

In practice, the different ingredients are mixed at the weight ratio shown in Table 3 above. The resulting composition, which is in liquid form, is submitted to sterilization (flash pasteurization) during 30 seconds at 124°C. This sterilization technic allows inactivating the bacteria while minimizing their disruption and respecting their integrity. As a result, the composition contains more than 80% intact cells and less than 20% damaged cells.

Such a composition is stored under aseptic conditions and further formulated in e.g. a cosmetic composition as disclosed below.

### II/ COSMETIC SOLUTION ACCORDING TO THE INVENTION

A composition according to the invention can be in the form of a solution containing the composition of the invention (A), as well as further cosmetically acceptable excipients. Such a formulation can have the composition disclosed in the Table 4 below:

| **Component** | **% (w/w)** |
|---|---|
| Purified water | 61,2 |
| Composition of the invention (A) | 1 |
| Glycerin | 8 |
| Solagum AX (Acacia Senegal Gum (and) Xanthan Gum) | 0,4 |
| Arlacel 2121 (Sorbitan Stearate (and) Sucrose Cocoate | 6 |
| Montanov 68 (Cetearyl Alcohol (and) Cetearyl Glucoside) | 1,65 |
| Emogreen L 15 (C15-19 Alkane) | 6 |
| Dub MCT 5545 (CAPRYLIC/CAPRIC TRIGLYCERIDE) | 3,2 |
| Cetiol LC (Coco-Caprylate/Caprate) | 2,25 |
| Huile de Bourrache (Borago Officinalis Seed Oil) | 2,8 |
| Beurre de Karité (Butyrospermum Parkii (Shea) Butter) | 2 |
| Huile de Cameline (Camelina sativa oil) | 2,35 |
| Coviox (Tocopherol) | 0,1 |
| Dermosoft 1388 (Glycerin (and) Aqua (and) Sodium Levulinate (and) Sodium Anisate) | 3 |
| Lactic acid (Lactic acid) | 0,05 |

### III/ BENEFICIAL EFFECTS OF A COMPOSITION ACCORDING TO THE INVENTION

### MATERIALS AND METHODS:

### 1/ Cellular models

### 1.1/ Normal Human Epidermal Keratinocytes (NHEKs)

The Normal Human foreskin-derived Epidermal Keratinocytes isolated from 3 Caucasian donors (NHEKs, Lonza, 00192906) were grown in Epilife medium (Fisher Scientific, M-EPI-500-A) supplemented with Human Keratinocyte Growth Supplement (HKGS, Fisher Scientific, S-001-5). The cells were maintained in a humidified incubator at 37°C with a 5% CO₂ atmosphere.

### 1.2/ Reconstituted Human epidermis (RHEs)

The epidermis were reconstituted *in vitro* with NHEKs keratinocytes (RHE, StratiCELL). The tissues were cultured at the air-liquid interface in Epilife medium (Fisher Scientific, M-EPI-500-A) containing supplements. They were maintained in a humid atmosphere at 37°C with 5% CO₂.

### 1.3/ Normal Human Dermal Fibroblasts (NHDFs)

The Normal Human foreskin-derived Dermal Fibroblasts (NHDFs; ATCC, CRL-2522) cells were grown in Dulbecco's Modified Eagle Medium (DMEM, Gibco/Life Technologies, 31885) supplemented with 10% of Foetal Bovine Serum (FBS, Gibco/Life Technologies, 10270). The cells were maintained in a humidified incubator at 37°C with a 5% CO₂ atmosphere.

### 2/ Staphylococcus epidermidis growth on 3D RHE

### 2.1/ RHE inoculation by S. epidermis

After 16 days at the air-liquid interface (D16), RHE were inoculated with *Staphylococcus epidermidis* (ATCC 12228, Fisher Scientific, 12999068, lot: 465973). *S. epidermidis* stored at - 80°C was initially plated on agar Trypsic Soy Brain (TSB + Agar = TSA, VWR, 20767.232) and incubated for one day at 37°C. Colonies were transferred into liquid medium TSB and grown at 37°C until mid-log phase. Bacteria were harvested to prepare an inoculum of 10³ CFU (Colony Forming Units)/RHE for bacterial growth monitoring in Epilife. Bacterial suspensions were added topically on the RHE for 24h. Test items were prepared in Epilife at a determined concentration of: 0.079% for B, 0.29% for E and 0.3% for A.

### 2.2/ Inoculum and sterility control

Serial dilutions were performed from inoculum and spotted on TSA-agar plates. After one day incubation at 37°C, colonies were counted and the number of CFU/mL (N) was determined using the following formula: N = n x (1/v) x (1/d) with:
n: mean number of colonies
v: deposited volume
d: dilution factor

Once the RHE inoculated with *S. epidermidis* and treated with B, E and A (at T0h), 20µL culture media were collected from each RHE and deposited on TSA-agar plate. After incubation at 37°C, sterility controls were checked. The same verification was done at the end of the experiment (T24h). Furthermore, all used products sterility was controlled.

### 2.3/ Bacterial growth evaluation

At the end of the contact period on the RHE *of S. epidermidis* and test compounds (B, E and A), bacteria were collected in PBS 0.1%-Tween80 with a swab and seeded on TSA agar plate (according to the decimal dilution method). After one day of incubation at 37°C, colonies were counted to determine the number of CFU.

### 3/ Measurement of ROS production

The detection and quantification of the intracellular ROS amounts from 2D NHEK keratinocytes or 3D RHE were performed using the H₂DCFDA (2'-7'dichlorodihydrofluorescein diacetate; *Fisher Scientific; D399*) probe. After its diffusion into the live cells, the probe is deacetylated by intracellular esterases to a non-fluorescent compound. This compound can be oxidized by ROS and converted into the highly fluorescent DCF (2'-7' dichlorofluorescein). The production of ROS by cells can be therefore measured through the level of fluorescence emitted by the oxidized dye.

### 3.1/ RHE treatment and ROS analysis

After 14 days of differentiation (D14), the test compounds were topically applied on RHE (3mg/cm²). In parallel, the effect of an anti-oxidant reference molecule (ascorbic acid 300µg/mL) was analyzed, by application in the culture medium for 24h. At day 15 (D15), RHE were rinsed, placed into fresh PBS, the reference and compounds were renewed and RHE were incubated with the H₂DCFDA probe during 40 min (at the concentration of 14,5µM in PBS). Then, RHE were irradiated with UVA rays at 30J/cm² (~ 1h20) to induce ROS production. Following the irradiation, RHE were transferred in a new culture plate and the fluorescence, directly related to the production of ROS by cells, was measured (excitation: 475 nm; emission: 500-550 nm) with a Glomax Explorer microplate reader (*Promega*). The data were normalized to cell metabolic activity which was measured by the MTS assay (3-(4,5-dimethythiazol-2-yl)-5-(3-carboxy-methoxyphenyl)-2-(4-sulfophenyl)-2H-tetrazolium), as described before.

### 3.2/ NHEK keratinocytes treatment and ROS analysis

NHEK keratinocytes in 24-well plates were pre-treated in the culture medium during 24h by the compounds at 1 concentration or with the antioxidant reference, ascorbic acid at 300µg/mL. NHEK keratinocytes were then placed in HBSS with the same test of reference treatments, in presence of the H₂DCFDA probe, during 30 min (15min at the concentration of 10µM and 15 min at those of 5µM) at 37°C and 5% CO2 atmosphere. Cultures were then rinsed and placed into fresh solution containing the test or reference compounds for the challenge with UVA radiations at 5J/cm² (Biosun, Vilber Lourmat, ~20min). Following the challenge, the fluorescence was measured (excitation: 475 nm; emission: 500-550 nm) with a fluorescence microplate reader (*Glomax Explorer, Promega*). The data of ROS production were normalized to cell metabolic activity (MTS assay).

### 4/ Quantification of pro-MMP1 and MMP1 activity by ELISAs assays

### 4.1/ NHDFs treatment

NHDF fibroblasts were cultivated on 24-well plates in complete culture medium (containing 10% FBS) 24h before the treatment with the compounds. Cells were then treated for 72h with the test compounds in culture medium, in absence of FBS. In parallel, reference treatments were applied according to the same method (TGF-β at 20ng/ml; combination of TNFα at 20ng/mL and IE1β at 2.5ng/mL). All the treatments were performed in triplicate of culture (n=3).

### 4.2/ ELISA assays

At the end of the 72h of treatments, all the supernatants were collected, aliquoted and stored at -20°C until further use in specific ELISA assays. Through these assays, the quantifications of the extracellular releases of pro-MMP1 (R&D systems, DMP100) produced by NHDFs fibroblasts were assessed according to the supplier's specifications.

### RESULTS:

### III-1/ GROWTH OF STAPHYLOCOCCUS EPIDERMIS

The objective of the test was to investigate the growth *of S. epidermidis,* the most abundant commensal bacterium on the skin, which participates in skin homeostasis by fighting pathogens and producing beneficial bacterial metabolites.

As shown on Figure 1, the extract alone (E) slightly decreased the growth *of S. epidermidis.* This can be explained by the antimicrobial properties of polyphenols as reported previously in relation to *S. aureus.* It should be noted that this same extract (E) did not show any deleterious effects on *L. paragasseri* CNCM 1-5892 (B). It is observed that the grape extract (E) and the *L.paragasseri* CNCM 1-5892 strain (B) that form the active composition according to the invention (A) increase the growth *of S. epidermidis* and that this growth is significant for the combination, which confirms this potential stimulation effect. The *L. paragasseri* CNCM 1-5892 strain of the invention thus appears to be a prebiotic that stimulates the growth of the commensal skin bacterium *S. epidermidis.*

### III-2/ ANTI-OXIDATIVE ACTIVITY

The objective of the test was to evaluate the antioxidant activity of the grape extract (E) and the active ingredient (A).

As shown on Figure 2, both the extract (E) and the active (A) have an antioxidant action with respect to a target molecule, ascorbic acid, on a population of monolayer keratinocytes (NHEK) subjected to UVA. The 0.29% extract and the 0.3% active ingredient according to the invention were tested for their potential antioxidant effect.

Exposure of NHEK to UVA radiation induced a very significant increase in ROS production and treatment with ascorbic acid partially suppressed this effect. These results validate the assay and analysis. Treatment with the extract very significantly reduced ROS production, at a level similar to vitamin C. In the presence the composition according to the invention, the effect was less important but significant.

### III-3/ INHIBITORY ACTIVITY ON PRO-MMP1

The results collected for NHDFs fibroblasts treated with test items and TGF-β1 20ng/ml (negative reference) or the combination TNFα 20ng/mL - IE1β 2.5ng/mL (positive reference) are illustrated in Figure 3. with the untreated condition set to 100%.

As expected, the negative (TGF- β1) and positive (TNFα + IE1β) references reduced and increased highly significantly the release of pro-MMP1 in supernatant of NHDFs fibroblasts cultures and validated the experiment.

The treatment with the composition according to the invention (A) at 0.03% reduced significantly the pro-MMP1 release compared to the untreated control.

Therefore, the active ingredient has an effect on the remodelling of the extracellular matrix as shown by measuring the abundance of the biomarker pro-MMP-1. The results show that the presence of the active ingredient significantly reduces the quantity of pro-MMP1, which is an interstitial collagenase that is involved in the degradation of fibrillar collagen.

## Claims

1. A composition comprising:
- an inactivated strain of lactobacillus; and
- an aqueous extract obtained from grape seeds and/or skins, advantageously from fermented red grapes.

2. The composition of claim 1 wherein the strain of lactobacillus belongs to the species *Lactobacillus paragasseri.*

3. The composition of claim 2, wherein the strain is the strain deposited at the CNCM (Collection Nationale de Cultures de Microorganismes, Institut Pasteur, 25 rue du Docteur Roux, 75724 Paris Cedex 15) under number 1-5892 on September 13, 2022.

4. The composition of any of claims 1 to 3, wherein the bacteria are inactivated by sterilization, advantageously by flash pasteurization.

5. The composition of any of claims 1 to 4, wherein the bacteria represent between 0.1 and 2% w/w, advantageously between 0.5 and 2% w/w of the composition.

6. The composition of any of claims 1 to 5, wherein the extract has been submitted to ultrafiltration after aqueous extraction.

7. The composition of any of claims 1 to 6, wherein the extract contains:
- tannins in a total amount advantageously representing 3 to 6 % w/w of dry matter;
- molecular tannins in an amount advantageously representing 0.05 to 0.15 % w/w of dry matter;
- crown tannins in an amount advantageously representing 0.005 to 0.015 % w/w of dry matter.

8. The composition of any of claims 1 to 7, wherein the aqueous extract represents between 4 and 8% w/w of the composition.

9. A method of preparing the composition of claims 1 to 8 comprising:
- Mixing the aqueous extract with the lactobacillus strain, advantageously at least 10⁸ CFU (Colony Forming Unit) of bacteria per ml of the extract; and
- Submitting the mixture to sterilization, advantageously by flash pasteurization.

10. The composition of any of claims 1 to 8, wherein it further comprises excipients, advantageously glycerin, and xanthan gum.

11. A composition of any of claims 1 to 8 and 10, wherein it is in the form of a cream, ointment, mask, serum, milk, lotion, paste, foam, aerosol, stick, deodorant, shampoo, conditioner, patch, aqueous-alcoholic or oily solution, oil-in-water or water-in-oil emulsion or multiple, an aqueous or oily gel, a liquid, pasty or solid anhydrous product, and/or a dispersion of oil in an aqueous phase with the aid of spherules, it being possible for these spherules to be polymeric nanoparticles such as nanospheres and nanocapsules or lipid vesicles of the ionic and/or non-ionic type.

12. A composition of any of claims 1 to 8 and 10 to 11 for use in cosmetics or in medicine.

13. A composition of any of claims 1 to 8 and 10 to 11 for use by topical administration.

14. A composition of any of claims 1 to 8 and 10 to 11 for use as an anti-ageing agent, an antioxidant and/or an anti-inflammatory agent.

15. A composition of any of claims 1 to 8 and 10 to 11 for use in the treatment of skin irritation and/or skin redness and/or skin hydration and/or skin barrier protection.
